# EUROPEAN PATENT SPECIFICATION

(11) **EP 0 504 409 B1**
(45) Date of publication and mention of the grant of the patent: **04.12.1996**
(21) Application number: 91913492.4
(22) Date of filing: 09.07.1991
(51) Int. Cl.: G01N 33/68

(54) **METHOD OF DIAGNOSING COMPLICATED UROLITHIASIS AND PROGNOSTICATING UROLITHIASIS**
VERFAHREN ZUR DIAGNOSE DER KOMPLEXEN UROLITHIASE UND PROGNOSE DER UROLITHIASE
PROCEDE POUR DIAGNOSTIQUER L'UROLITHIASE COMPLEXE ET PRONOSTIQUER L'UROLITHIASE

(30) Priority: 08.08.1990 SU 4858034; 11.10.1990 SU 4873890
(43) Date of publication of application: 23.09.1992
(73) Proprietor: SHABALIN, Vladimir Nikolaevich, Moscow, 129301 (SU); SHATOKHINA, Svetlana Nikolaevna, Moscow, 129041 (SU)
(72) Inventor: SHABALIN, Vladimir Nikolaevich, Moscow, 129301 (SU); SHATOKHINA, Svetlana Nikolaevna, Moscow, 129041 (SU); DUTOV, Valery Viktorovich, Moscow, 117513 (SU); TRAPEZNIKOVA, Margarita Fedorovna, Moscow, 117311 (SU); MOROZOV, Andrei Petrovich, Moscow, 117331 (SU); Miroshnikov, Andrei Nikolaevich, Moscow (SU); Makshin, Leonid Georgievich, Moscow (SU); Yakovlev, Sergei Anatolievich, korpos 1, kv.77. (SU)
(74) Representative: Allden, Thomas Stanley
(86) International application number: SU9100140
(87) International publication number: WO9202821

(56) References cited:
- WO-A-93/23753
- DE-A- 3 643 263
- SU-A- 607 141
- SU-A- 1 337 777
- SU-A- 1 573 425
- SU-A- 1 629 846
- NEPHROLOGIE vol. 6, no. 5 , 1985 , BASEL CH pages 228 - 230 P. DEQUIEDT ET AL. 'Acute oxatose manifesting as acute renal failure after a masive ingestion of piridoxilate.'
- V.E. PREDTECHENSKY "Rukovodstvo po clinicheskim loboratornym issledovaniam", izdanie shestoe, 1964, Meditsina, pages 427, 444 (cited in the description).

## Description

The invention relates to medicine, and more specially, to methods of diagnosing and prognosticating complicated urolithiasis.

At present, for the purposes of diagnosing urolithiasis, particularly calculous pyelonephritis, use is made of clinical laboratory examination of urine, the X ray and ultrasound analysis of kidneys and urine excretory tracts.

A possibility of the onset of urolithiasis is evaluated if risk factors are available such as hereditary predisposition and endemicity.

Known in the art is a method of diagnosing pyelonephritis based on laboratory methods of investigations, one of which is determination of the contained albumin in urine. The method is based on coagulation of the albumin with chemical reagents, for example, nitric acid, sulfosalicyclic acid, to mention only a few; physical methods, particularly, heating said urine etc (Spravochnik po klinicheskim laboratornym metodam issledovaniya. Edited by E. Kost, Moscow, MEDITSINA Publishers, 1975; Laboratornye metody issledovaniya v klinike. Spravochnik, edited by V. Menshikov, Moscow, MEDITSINA Publishers, 1987).

Also known is a method for the examination of a urine sample to determine whether protein is present in the sample, the method being based on crystallography. It consists in the following: a sample from the top layer of settled urine is applied to a microscope slide and dried to complete crystallisation; the presence of protein is established in the urine under examination, when a marginal amorphous area is apparent. When no crystallisation at all is observed, it is concluded that the urine sample contains glucose (SU-A1-1573625). The method can be used in accordance with the present invention for diagnosing pyelonephritis.

Known in the art is a method for diagnosing urinary calculous disease which is based on finding a process of calculus formation in cases where a precipitate is observed in freshly excreted urine or a short while after urination (V. Predtechensky "Rukovodstvo po Klinicheskim laboratornym issledovaniyam", MEDITSINA, 1964, pp.420-446). Such examinations are performed in native urine. In order to slow down evaporation and dessication, a urine sample is covered with cover glass.

Also known is a method for prognosticating urolithiasis according to which determination is given to a degree of risk of calcium phosphate crystallisation in urine by counting the formed crystals of specific size according to mathematical calculations with the account taken of certain factors ("Urological Research", N 2, 15,1987, Springer-Verlag, H.-G. Tiselius "Measurement of the Risk of Calcium Phosphate Crystallisation in Urine", s. 79-81).

Also known is a method for prognosticating urolithiasis which allows for a combined calculation of the morphological parameters of cuppelvic system with the clinical-chemical parameters of urine on the basis of a discriminant analysis with consideration of eight functions for 3-10 parameters ("European Urology", 16, N 3, 1989, S. Karger A.G. Basel; E. Schultz, R. Boerner, P. Brundig, F. Maeurer "Influence of Different Factors on the Formation of Calcium Oxalate Stone", s.218-222).

It is the principal object of the present invention to specify the diagnosis of complicated urolithiasis, more specifically, calculous pyelonephritis, and also to predict said urolithiasis in "healthy" persons, when urine examination shows no deviations from the normal situation and subjective complaints are absent.

The above objective is accomplished by means of the claimed new method of diagnosing these diseases. It consists in that alongside the clinical examination of a urine sample for the presence of protein, a portion of the urine sample is applied to the surface of a glass substrate, and dried, followed by crystallographic examination. The presence in the dried portion of said sample of a marginal amorphous area, within which the central crystalline area is located, points to the presence of pyelonephritis. And, if said amorphous area is absent, i.e. inspection reveals a full crystalline area, the diagnosis calculous pyelonephritis is drawn.

The claimed invention enables simple diagnosis in clinical laboratory practice requiring no X-ray or ultrasound examination, a factor that enables one to avoid radiation and ultrasound treatment of the patient and also avoids the use of expensive and difficulty available apparatuses. The method can be carried out easily by a skillful examiner.

Furthermore, for predicting urolithiasis in healthy persons, the following method is given. It consists in that alongside the clinical examination of urine samples for absence of protein, 8-12% protein is added, e.g. albumen. A sample is applied to a glass substrate and is dried at room temperature for about 24 hours and subsequently, crystallographic investigations are performed. With the appearance of a full non-transparent crystallization, i.e. no amorphous area is present, prognostication of urolithiasis is determined.

Timely dietary measures and medicamentous therapy can prevent a process of stone formation in such patients. The claimed method contributes to prognosticating urolithiasis in persons having not only hereditary predisposition but also any forms of acquired urolithiasis, i.e. actually 100% of cases.

The claimed method assures the following advantages: to predict urolithiasis rapidly, without a patient's pre-preparation and without using special equipment and expensive reagents.

The method can be introduced into practice on a wide scale in all medical establishments and provides a possibility of mass-scale examinations during preventive treatment directed to prevention of the progress of urinary calculous disease.

The invention is explained by a clear description of its dependent features of realization with reference to the accompanying drawings in which:

Fig. 1 shows a marginal amorphous area accompanying a central crystalline area in a positive protein sample of urine. Conclusion: pyelonephritis of non-calculous origin.

Fig. 2 shows a full crystalline area, in other words, no marginal amorphous area is present, which indicates, in the case of a presence of protein in the urine sample, pyelonephritis of calculous origin, is diagnosed.

Fig. 3 shows a marginal amorphous area accompanying a central crystalline area, after crystallisation of a mixture of urine and protein, where the urine sample itself does not contain protein. Conclusion: no diagnosis or prognosis of urolithiasis.

Fig. 4 shows a full crystalline area and the absence of a marginal amorphous area after crystallisation of a mixture of urine and protein, where the urine sample itself does not contain protein. Conclusion: urolithiasis is prognosticated.

Preferably, the claimed method of diagnosing complicated urolithiasis, particularly calculous pyelonephritis, and pyelonephritis of non-calculous origin is carried out in the following manner.

Urine is sampled and subjected to clinical laboratory examination for the presence of protein therein. When protein is found in the urine sample, a portion of the urine sample/0,01 - 0.02/ is applied to the surface of a microscope slide and allowed to dry at room temperature to complete dryness (30-40 minutes). The dried urine sample is subjected to visual crystallographic study, followed by the performance of a comparative analysis of crystallisation of the urine sample and a qualitative reaction for protein, say, in a test tube.

### Example 1.

Patient A. On visual crystallographic examination two areas are discovered: a central crystalline area and, on the margin thereof, an amorphous area. The urine sample itself contains protein. Conclusion: pyelonephritis of non-calculous origin.

Clinical diagnosis: chronic pyelonephritis (cf Fig.I).

### Example 2.

Patient V. Crystallographic examination reveals one crystalline area. The urine sample itself contains protein.

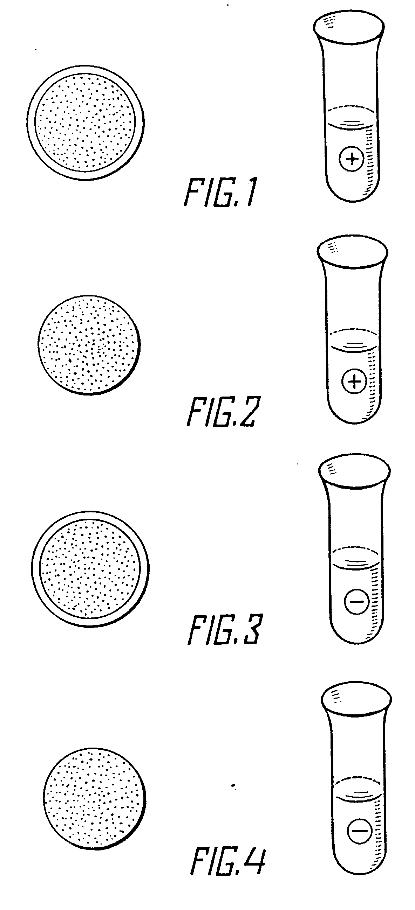

Conclusion: calculous pyelonephritis (cf. Fig.2).

Clinical diagnosis: calculous. Secondary pyelonephritis.

The claimed method was used for verifying the presence of protein in 61 samples in a range of concentrations of from 0,033 g/l to 0,495 g/l.

The claimed method makes it possible to perform a differential diagnosis of pyelonephritis and calculous pyelonephritis and is substantiated by the fact that in some pyelonephritis cases, etiology may be "calculous". This method works in 100% of cases and must be carried out at the very first stages of the patients' examination.

The method of diagnosis was verified on 36 patients. Results are given in Table I.

The claimed method for the prognostication of urolithiasis is carried out as follows:

An excreted urine portion is taken from a healthy person and subjected to a clinical laboratory examination to confirm the absence of protein. Furthermore, a part of the top layer of a settled urine sample is removed and an 8-12% protein (for example albumen) solution is added. Said mixture having a volume of 20-50 µl is then applied to a glass substrate and allowed to stay at room temperature for 24 hours.

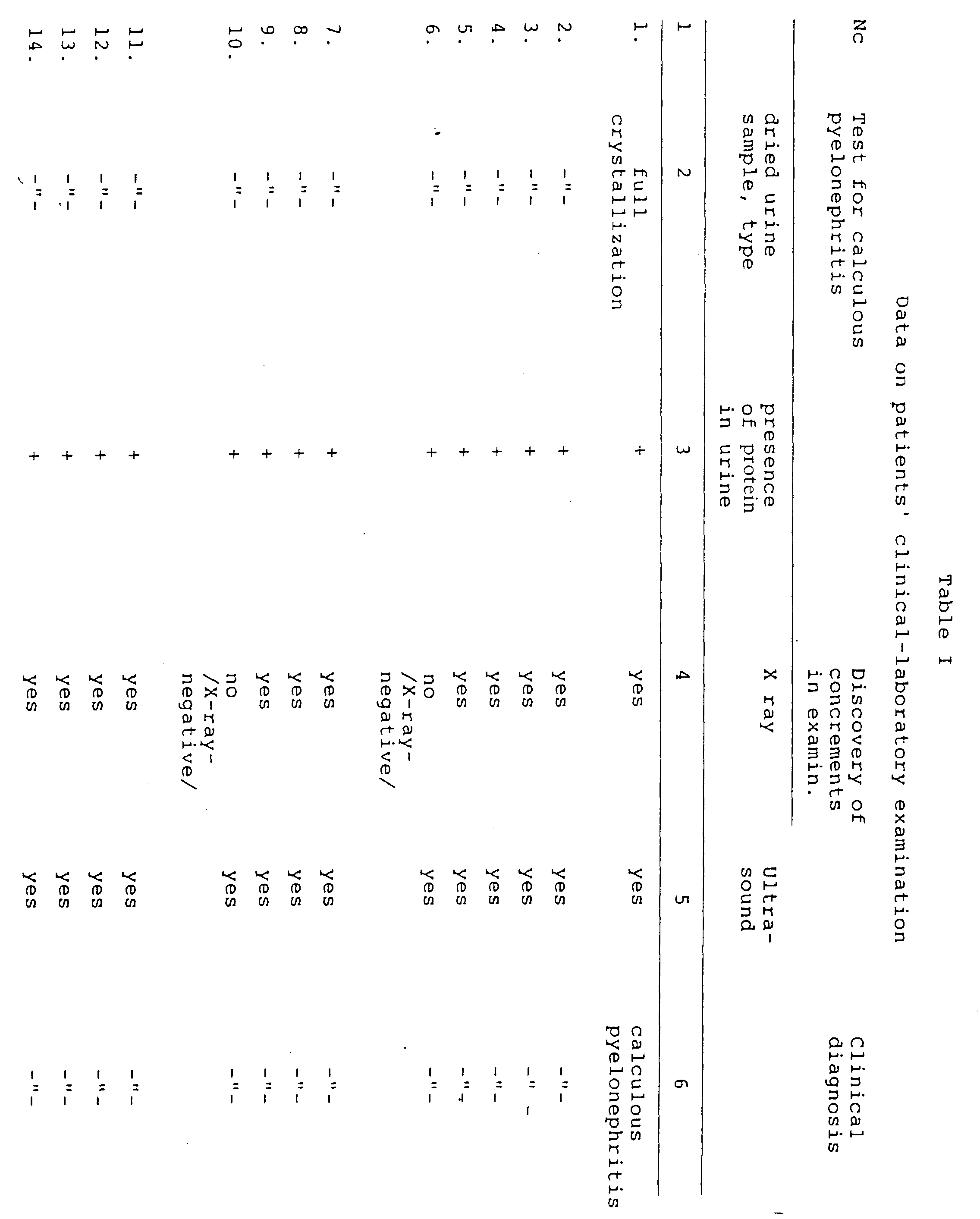

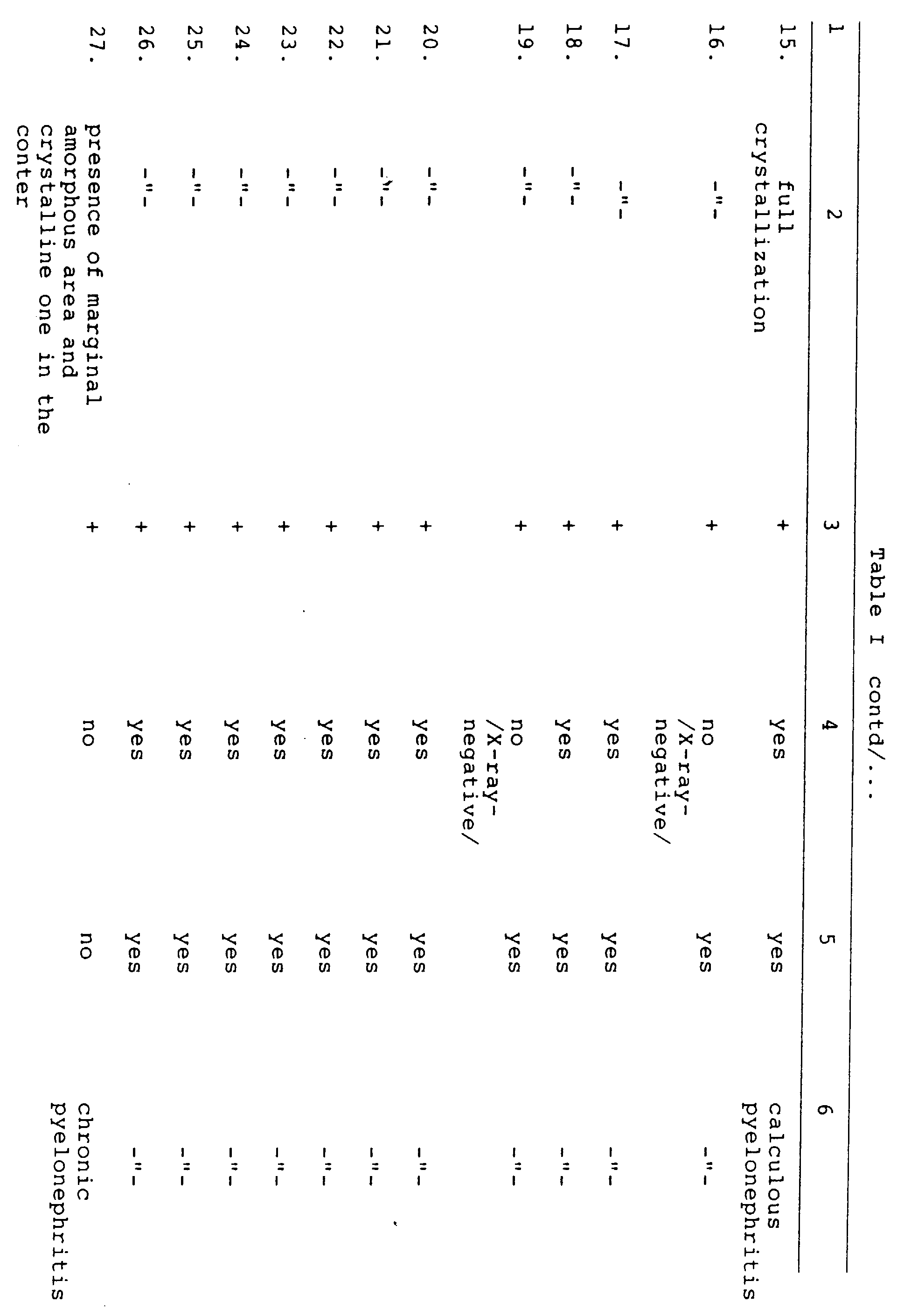

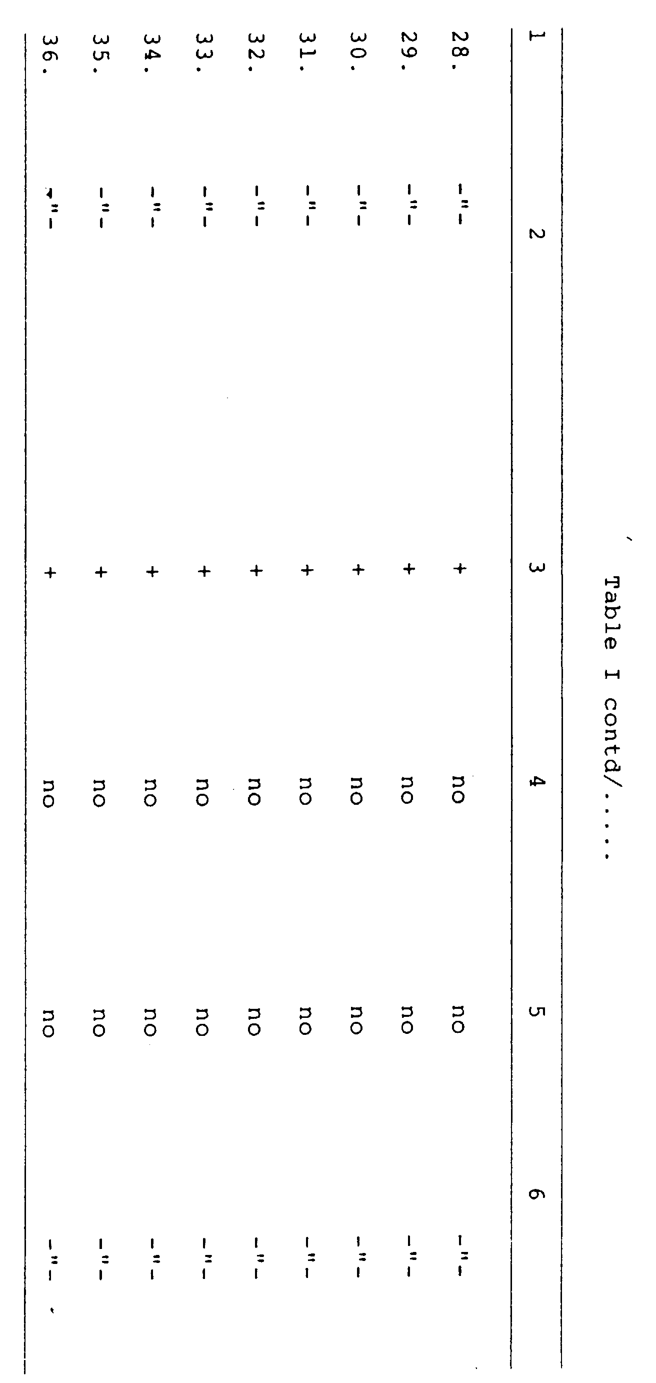

By way of control, a portion of the urine sample is applied to the glass substrate, without adding protein. The dried mixture of urine and protein is subjected to visual crystallographic examination.

Following this, patterns of crystallisation are studied for the presence of central and marginal areas.

### Example 3.

Patient S. On crystallographic examination of a mixture of urine and protein two areas can be observed: a central crystalline area and, on the margin thereof, a transparent amorphous area. The urine sample itself does not contain protein.

Conclusion: no prognostication of urolithiasis.

Clinical diagnosis: right kidney prolapse (cf. Fig.3).

### Example 4.

Patient Di. Crystallographic examination of a mixture of urine and protein reveals one area that is completely crystallised.

Conclusion: urolithiasis is prognosticated.

Clinical diagnosis: nephrolithiasis (cf. Fig.4).

It is noteworthy that in the two above-described examples 3 & 4, the control samples provided one and the same picture, namely, one completely crystalline area, i.e. no result regarding the prediction of the disease is obtained.

The claimed method enables one to prognosticate urolithiasis in healthy persons and take measures towards preventing a process of stone formation. The method is simple, and easy in realisation.

The method was checked on 108 practically healthy people. Results are given in Tables 2 and 3.

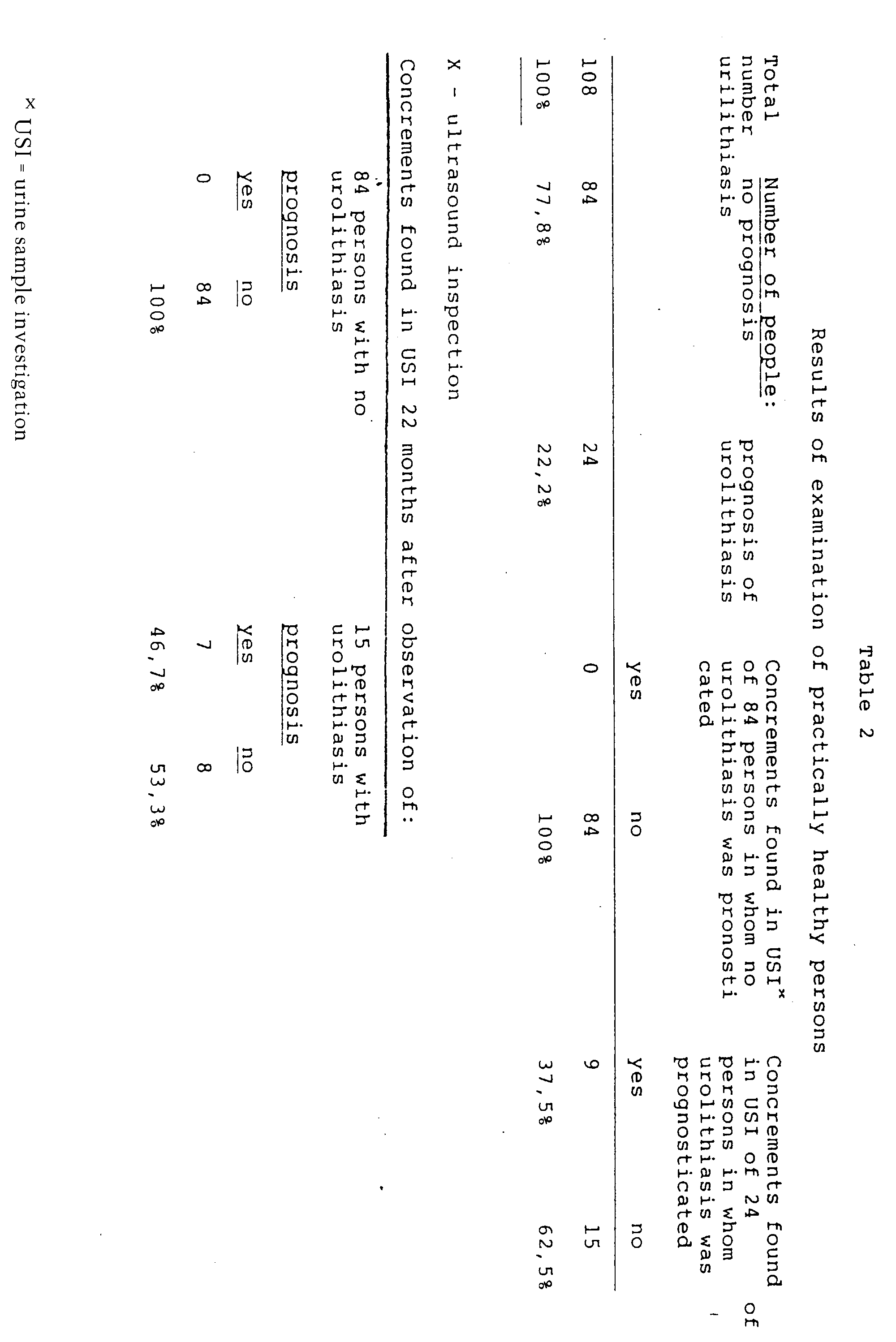

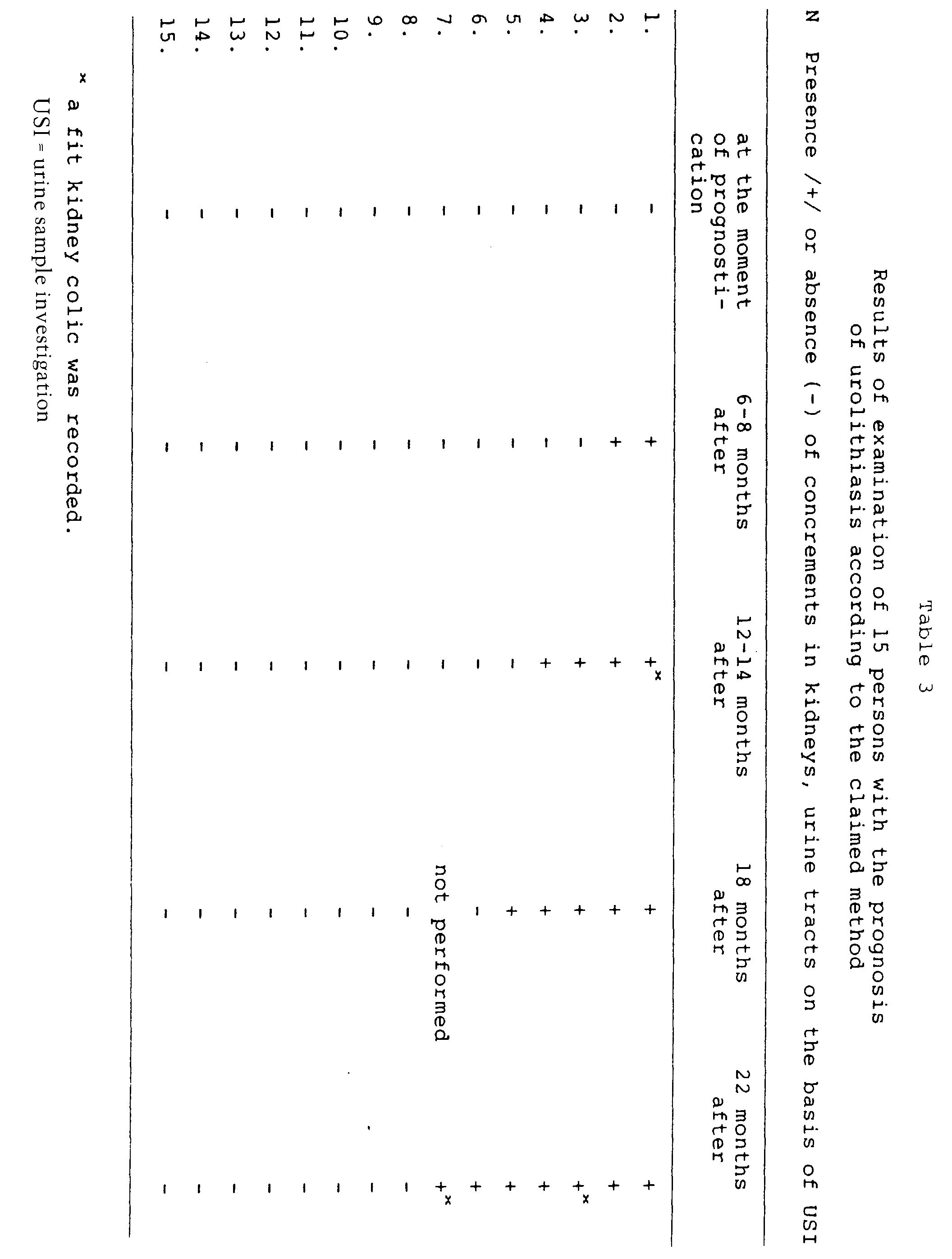

The invention may find a variety of applications in the practice of all medical establishments because it does not require expensive facilities and reagents. It is advisable to put this invention into practice during mass-scale prophylactic examinations of population for purposes of carrying out preventive treatment directed to prevention of the progress of urolithiasis.

## Claims

1. A method of prognosticating urolithiasis using a urine sample which does not contain protein, wherein there is added to a portion of the urine sample an 8-12% solution of a protein, such as albumen, the mixture is dried on a glass substrate at room temperature for 24 hours, followed by a crystallographic examination, urolithiasis being prognosticated with the full non-transparent crystallisation of the sample, with no peripheral amorphous area.

2. A method of diagnosing complicated urolithiasis, which method comprises testing a urine sample for the presence of protein, and, only if protein is present, drying a portion of the urine sample containing protein on a glass substrate, followed by a crystallographic examination, wherein complicated urolithiasis is diagnosed with the full non-transparent crystallisation of the sample, with no peripheral amorphous area.

3. A method of diagnosing non-calculous pyelonephritis, which method comprises testing a urine sample for the presence of protein, and, only if protein is present, drying a portion of the urine sample containing protein on a glass substrate, followed by a crystallographic examination, wherein non-calculous pyelonephritis is diagnosed if a peripheral amorphous area and a central crystalline area are observed.

## Patentansprüche

1. Methode zur Prognostizierung der Urolithiasis unter Zuhilfenahme einer Harnprobe, welche kein Protein enthält, wobei einem Teil der Harnprobe eine 8-12%ige Lösung eines Proteins, beispielsweise eines Eiweißstoffes, zugesetzt wird, die Mischung auf einem Glassubstrat bei Zimmertemperatur 24 Stunden getrocknet wird, gefolgt von einer kristallographischen Untersuchung, wobei Urolithiasis bei vollständig undurchsichtiger Kristallisation der Probe ohne amorphen Randbereich prognostiziert wird.

2. Methode zur Diagnostizierung komplizierter Urolithiasis, wobei die Methode folgendes umfaßt: Untersuchung einer Harnprobe auf Anwesenheit von Protein und ausschließlich bei Vorhandensein von Protein, Trocknen eines Teils der proteinhaltigen Harnprobe auf einem Glassubstrat, gefolgt von einer kristallographischen Untersuchung, wobei bei vollständiger undurchsichtiger Kristallisation der Probe ohne amorphen Randbereich eine komplizierte Urolithiasis diagnostiziert wird.

3. Methode zur Diagnostizierung einer nicht konkrementären Pyelonephritis, wobei die Methode folgendes umfaßt: Untersuchung einer Harnprobe auf das Vorliegen von Protein und ausschließlich bei Vorliegen von Protein, Trocknen eines Teils der proteinhaltigen Harnprobe auf einem Glassubstrat gefolgt von einer kristallographischen Untersuchung, wobei bei der Feststellung eines amorphen Randbereichs und eines zentralen kristallinischen Bereichs eine nicht konkrementäre Pyelonephritis diagnostiziert wird.

## Revendications

1. Une méthode de pronostic des urolithiases qui utilise un prélèvement d'urine ne contenant pas de protéine, méthode selon laquelle il est ajouté, à une portion du prélèvement d'urine, une solution à 8-12% d'une protéine telle que l'albumen, le mélange étant séché sur un substrat en verre à la température ambiante durant 24 heures, puis examiné par analyse cristallographique, l'urolithiase étant pronostiquée avec la cristallisation complète non transparente du prélèvement, sans zone amorphe périphérique.

2. Une méthode de diagnostic des urolithiases compliquées, ladite méthode comportant l'analyse d'un prélèvement d'urine aux fins de détection de la présence de protéine et, uniquement en présence d'une protéine, de séchage d'une portion du prélèvement d'urine contenant la protéine sur un substrat en verre, le séchage étant suivi d'une analyse cristallographique, méthode selon laquelle une urolithiase compliquée est diagnostiquée avec la cristallisation complète non transparente du prélèvement, avec aucune zone amorphe périphérique.

3. Une méthode de diagnostic de pyélonéphrite non calculeuse, ladite méthode comportant l'analyse d'un prélèvement d'urine aux fins de détection de la présence de protéine et, uniquement en présence d'une protéine, de séchage d'une portion du prélèvement d'urine contenant la protéine sur un substrat en verre, le séchage étant suivi d'une analyse cristallographique, méthode selon laquelle une pyélonéphrite non calculeuse est diagnostiquée si l'on observe une zone amorphe périphérique et une zone cristalline centrale.
